(19) Europäisches Patentamt
European Patent Office
Office européen des brevets

(11) **EP 3 996 619 B1**

(12) # EUROPÄISCHE PATENTSCHRIFT

(45) Veröffentlichungstag und Bekanntmachung des Hinweises auf die Patenterteilung:
**30.08.2023   Patentblatt 2023/35**

(21) Anmeldenummer: **20739315.8**

(22) Anmeldetag: **07.07.2020**

(51) Internationale Patentklassifikation (IPC):
***A61B 18/26*** (2006.01)

(52) Gemeinsame Patentklassifikation (CPC):
**A61B 18/26; A61B 1/00006; A61B 1/12;**
**A61B 1/128; A61B 1/307;** A61B 18/22;
A61B 2018/00642; A61B 2018/0066;
A61B 2018/00678; A61B 2018/00791;
A61B 2018/00863; A61B 2018/263

(86) Internationale Anmeldenummer:
**PCT/EP2020/069125**

(87) Internationale Veröffentlichungsnummer:
**WO 2021/005060 (14.01.2021 Gazette 2021/02)**

(54) **SYSTEM ZUR TEMPERATURÜBERWACHUNG BEI DER DURCHFÜHRUNG EINER LASERLICHT-BASIERTEN INTRAKORPORALEN LITHOTRIPSIE**

SYSTEM FOR MONITORING TEMPERATURE WHILE INTRACORPORAL LASER LITHOTRIPSY IS BEING CARRIED OUT

SYSTÈME DE SURVEILLANCE DE LA TEMPÉRATURE PENDANT LA RÉALISATION D'UNE LITHOTRITIE ENDOCORPORELLE À LA LUMIÈRE LASER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.07.2019   DE 202019103823 U**

(43) Veröffentlichungstag der Anmeldung:
**18.05.2022   Patentblatt 2022/20**

(73) Patentinhaber: **Albert-Ludwigs-Universität Freiburg**
**79098 Freiburg (DE)**

(72) Erfinder:
- **MIERNIK, Arkadiusz**
  **79098 Freiburg (DE)**
- **HEIN, Simon**
  **79098 Freiburg (DE)**
- **PETZOLD, Ralf**
  **79098 Freiburg (DE)**

(74) Vertreter: **Rösler, Uwe**
**Rösler Patentanwaltskanzlei**
**Landsberger Strasse 480a**
**81241 München (DE)**

(56) Entgegenhaltungen:
**DE-U1-202017 102 316     US-A1- 2018 055 568**

**Beschreibung**

**Technisches Gebiet**

Die Erfindung bezieht sich auf ein System zur Temperaturüberwachung bei der Durchführung einer laserlichtbasierten intrakorporalen Lithotripsie, bei der eine Endoskopanordnung zum Einsatz kommt, die einen Arbeitskanal für einen Lichtleiter, der proximalseitig mit einem Laser optisch gekoppelt ist und distalseitig eine Lichtaustrittsapertur aufweist, sowie einen Spülflüssigkeitskanal umfasst, der im Bereich der Lichtaustrittsapertur distalseitig offen mündet und proximalseitig mit einem Spülflüssigkeitsreservoir fluidisch verbunden ist.

**Stand der Technik**

Die intrakorporale Lithotripsie betrifft eine minimalinvasive chirurgische Methode zur Zertrümmerung von Steinkonkrementen, beispielsweise Gallen-, Harn- oder Nierensteine, die sich vornehmlich in Ausführungsgängen der betreffenden Organe ansammeln und starke Schmerzen sowie andere medizinischen Probleme verursachen können. Unter den diversen bekannten intrakorporalen Lithotripsieverfahren hat sich die laserinduzierte Lithotripsie etabliert, bei der energiereiche Laserstrahlung über eine Lichtleitfaser an den Ort eines zu zertrümmernden Steines in Form von Laserpulsen appliziert wird. Durch Wechselwirkung der Laserpulse mit dem intrakorporalen Medium bildet sich eine Dampfblase aus, durch deren pulsartige Ausdehnung Druckwellen entstehen, die zur Fragmentation des Steins führen. Die bei der laserinduzierten Lithotripsie entstehenden Steinfragmente können in Abhängigkeit ihrer Größe auf natürlichem Wege ausgeschieden oder mit geeignet ausgebildeten endoskopischen Greifwerkzeugen extrahiert werden.

Zur Durchführung der laserbasierten Lithotripsie kommen Endoskope zum Einsatz, die wenigstens einen Arbeitskanal für einen Lichtleiter vorsehen, der proximalseitig mit einem Laser optisch gekoppelt ist und distalseitig eine Lichtaustrittsapertur aufweist. Um den organischen Zugängen zu den Steinen mit dem Endoskop folgen zu können und den Patienten während der Behandlung möglichst wenig zu belasten, können derartige Endoskope biegsam und flexibel gestaltet werden. Zudem kann man über derartige Endoskop eine Spülflüssigkeit meistens über einen Arbeitskanal applizieren, der im Bereich der Lichtaustrittsapertur distalseitig mündet und proximalseitig mit einer Spülflüssigkeitsfördereinrichtung, vorzugsweise in Form einer Spülspritze und/oder einer steuer- oder regelbaren Förderpumpe, sowie einem Spülflüssigkeitsreservoir fluidisch verbunden ist.

Mit Hilfe einer derartigen Endoskopanordnung, umfassend das Endoskop und die daran angeschlossenen, vorstehend erwähnten Peripheriegeräte, gilt es die intrakorporal am Ort des zu zertrümmernden Steins applizierte Laserleistung sowie die Menge der Spülflüssigkeit, die distalseits zum Spülkanal ausgetragen wird, derart aufeinander abzustimmen, dass zum einen eine effektive Steinzertrümmerung erfolgen kann, und zum anderen keine laserlichtbedingte lokale Überhitzung von umliegenden Gewebebereichen auftritt, die zu irreversiblen Gewebeschädigungen führen kann. Zumeist verfügen derartige Endoskope über eine zusätzliche Glasfaseroptik für den Operateur oder einen bildgebenden Sensor an der Endoskopspitze, den es mit Hilfe einer dosierten Spülflüssigkeitszuführung optisch freizuhalten gilt, um stets einen freien Blick für den Operateur zur Überwachung des Zertrümmerungsprozesses zu gewährleisten.

Die Druckschrift US 2018/0055568 A1 beschreibt eine gattungsgemäße Endoskopanordnung zur Durchführung einer laserbasierten Lithotripsie, deren zugeordnete Spülflüssigkeitsfördereinheit in Abhängigkeit wenigstens eines den Lithotripsieprozess beeinflussenden Parameters regelbar ist, d.h. die Spülrate wird in Abhängigkeit des Lithotripsieprozesses beeinflusst. Neben einer optischen Überwachung des Zertrümmerungsvorganges, sieht die bekannte Endoskopanordnung einen am distalen Ende des Endoskops angebrachten Temperatursensor vor, der die distalseitige Temperatur der ihn umgebenden Flüssigkeitzu erfassen vermag und die der Spülflüssigkeitsfördereinheit zur Einstellung der Spülflüssigkeitsrate zugrunde gelegt wird.

Auch bei dem bekannten medizinischen Endoskop gemäß der Druckschrift DE 20 2017 102 316 U1, das zur Durchführung einer laserbasierten Lithotripsie geeignet ist, ist zu Zwecken einer Temperaturmessung ein Umgebungstemperatursensor am distalen Bereich des Endoskops angeordnet.

Derartige mit Sensoren ausgestattete Endoskopanordnungen stellen hochkomplexe medizinische Instrumente dar, die einer langwierigen und umfangreichen medizinisch technischen Zulassungsprüfung unterliegen.

**Darstellung der Erfindung**

Der Erfindung liegt die Aufgabe zugrunde, ein System zur Temperaturüberwachung bei der Durchführung einer laserlichtbasierten intrakorporalen Lithotripsie, bei der eine Endoskopanordnung zum Einsatz kommt, die einen Arbeitskanal für einen Lichtleiter, der proximalseitig mit einem Laser optisch gekoppelt ist und distalseitig eine Lichtaustrittsapertur aufweist, sowie einen Spülflüssigkeitskanal umfasst, der im Bereich der Lichtaustrittsapertur distalseitig mündet und proximalseitig mit einem Spülflüssigkeitsreservoir fluidisch verbunden ist, derart weiterzubilden, so dass eine überwachte intrakorporale Lithotripsiedurchführung auch mit gattungsgemäßen Standard Endoskopen möglich wird, ohne geld- und zeitraubende Aufwendungen leisten zu müssen, die in Zusammenhang mit einer medizinisch technischen Zulassungsprüfung bzw. Zertifizierung verbunden sind. Es gilt nach Maßnahmen zu suchen, mit denen bereits im Einsatz befindliche Endoskope, Lasersysteme und weitere medizinisch-technische Vorrichtungen, wie beispielsweise Spülflüssigkeitsvorrichtungen zur passiven und aktiven Spülung die zur laserbasierten Lithotripsie grundsätzlich geeignet ausgebildet sind, mit geeignet gewählten Komponenten nachzurüsten, um mögliche thermisch induzierte Gewebedegradationen im unmittelbaren Umfeld am Ort der Steinzertrümmerung sicher ausschließen zu können.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Generell ist die intrakorporale Laserapplikation ohne Spülung, d.h. ohne jegliche Spülflüssigkeitsversorgung am Bereich der distalseitigen Lichtaustrittsapertur des Lichtleiters, nur kurzfristig, d.h. innerhalb weniger Sekunden möglich, zumal ohne jegliche spülungsbedingte Kühlung eine kritische Temperatur von ca. 42°C erreicht und überschritten wird, ab der das umliegende Gewebe thermisch irreversibel geschädigt wird. Demzufolge ist eine Spülung während der laserlichtbasierten Lithotripsie unverzichtbar, um eine ausreichende Kühlung zu gewährleisten.

Anhand einer großen Vielzahl durchgeführter Versuche zur laserbasierten Lithotripsie konnte ein belastbarer, numerischer bzw. theoretischer Zusammenhang zwischen der intrakorporal applizierten Laserleistung, der Spülflüssigkeitsrate, der Temperatur der Spülflüssigkeit und einer sich intrakorporal während der Laserpulsapplikation einstellenden Temperatur gefunden werden.

Auf der Basis dieser Erkenntnis eröffnet sich die Möglichkeit, an bekannte Endoskope mit wenigstens einem Arbeitskanal für einen Lichtleiter, der proximalseitig mit einem Laser optisch gekoppelt ist und distalseitig eine Lichtaustrittsapertur aufweist sowie einen Spülflüssigkeitskanal umfasst, der getrennt zum oder einheitlich mit dem Arbeitskanal ausgebildet sein kann, und im Bereich der Lichtaustrittsapertur distalseitig offen mündet und proximalseitig mit einer optionalen Spülflüssigkeitsfördereinheit und einem Spülflüssigkeitsreservoir fluidisch verbunden ist, mit einer lösungsgemäß ausgebildeten Moduleinheit in der folgenden Weise zu kombinieren: Die Moduleinheit umfasst wenigstens einen Durchflusssensor sowie wenigstens einen Temperatursensor, die jeweils längs des Spülflüssigkeitskanals und/oder einer mit dem Spülflüssigkeitskanal fluidisch verbundenen Zuleitung, vorzugsweise lösbar fest, längs des extrakorporalen Bereich der Endoskopanordnung anbringbar sind. Der Durchflusssensor ist derart ausgebildet und angeordnet, dass die Spülflussrate kontaktfrei zur Spülflüssigkeit ermittelbar ist. Gleichsam ist der Temperatursensor derart ausgebildet und längs des Spülflüssigkeitskanals und/oder der mit dem Spülflüssigkeitskanal fluidisch verbundenen Zuleitung angebracht, dass die Spülflüssigkeitstemperatur kontaktfrei ermittelbar ist.

Ferner umfasst die Moduleinheit entweder ein Eingabemittel, über das Betriebsparameter des Lasers eingebbar und einer mit dem Eingabemittel verbundenen Rechnereinheit übermittelbar sind, oder eine Schnittstelle, über welche die Betriebsparameter des Lasers direkt übertragen werden können. Die Betriebsparameter des Lasers betreffen vorzugsweise die Laserleistung, wobei über die Laserpulsfrequenz sowie die Einzelpulsenergie die Berechnung einer am Ort der Lichtaustrittsapertur applizierten Laserleistung möglich ist.

Innerhalb der Rechnereinheit ist zudem eine Auswerteeinheit implementiert, die auf der Grundlage der sensorisch erfassten Spülflussrate, der sensorisch erfassten Temperatur der Spülflüssigkeit sowie der ermittelten intrakorporal applizierten Laserleistung eine sich während der laserlichtbasierten Lithotripsie intrakorporal am Ort der Lichtaustrittsapertur ausbildende Temperatur numerisch ermittelt. Getrennt zur oder integriert in der Rechnereinheit ist eine Komparatoreinheit angeordnet, die die ermittelte Temperatur mit einem einstellbaren Schwellwert vergleicht, bspw. 42 °C, und bei Überschreiten des Schwellwertes ein Signal erzeugt.

In einer bevorzugten Ausführungsform ist zudem eine Signaleinheit vorgesehen, die kabellos oder kabelgebunden mit der Komparatoreinheit verbunden ist und durch das von der Komparatoreinheit erzeugte Signal haptisch, akustisch und/oder visuell wahrnehmbar in Erscheinung zu treten vermag. Durch die Signaleinheit wird der die Lithotripsie durchführende Arzt vor möglichen intrakorporalen Überhitzungseffekten gewarnt, um auf diese Weise die Lithotripsiebehandlung zu unterbrechen oder anderweitige Maßnahmen zu treffen.

Die lösungsgemäße, vorzugsweise als Nachrüstsatz oder Nachrüstkit ausgebildete Moduleinheit umfasst ausschließlich Komponenten, die modulartig an eine im Einsatz befindliche Endoskopanordnung anbringbar sind, ohne die grundsätzliche Funktionalität der Endoskopanordnung zu beeinträchtigen. Das lösungsgemäße System stellt somit ein den Arzt bei der Durchführung einer laserbasierten Lithotripsie unterstützendes Sicherheitssystem dar, über das der Arzt zuverlässige und belastbare Informationen erhält, um den Lithotripsieprozess effektiv und patientenschonend durchführen zu können.

Die einzigen hierfür erforderlichen Informationen betreffen die aktuelle Temperatur der über die Endoskopanordnung vermittels des Arbeitskanals oder des Spülflüssigkeitskanals intrakorporal eingebrachte Spülflüssigkeit, die am Ort des zu zertrümmernden Steines applizierte Laserleistung sowie die Spülrate, mit der die Spülflüssigkeit durch den Arbeitskanal oder Spülflüssigkeitskanal intrakorporal am Ort der Laserlichtapplikation eingebracht wird.

Die für die Spülflüssigkeitstemperatur sowie Spülrate erforderlichen Sensoren stellen jeweils lösbar fest an den Spülflüssigkeitskanal und/oder an die mit dem Spülflüssigkeitskanal fluidisch verbundenen Zuleitung anbringbare Bauelemente dar, die in einem Bereich der Endoskopanordnung angebracht sind, der nicht intrakorporal in den Körper eingebracht wird. So eignet sich in bevorzugter Weise als ein die Spülflussrate bestimmender Sensor ein Durchflusssensor in Art eines Ultraschallsensors. Zur Erfassung der Spülflüssigkeitstemperatur eignet sich ein Temperatursensor in Art eines Infrarotsensors.

Zur Ermittlung der sich am Ort der Lichtaustrittsapertur des Lichtleiters ausbildenden Temperatur sind die am Laser vorgebbaren Betriebsparameter betreffend die Laserpulsfrequenz sowie die Einzelpulsenergie erforderlich. Diese Betriebsparameter sind über ein geeignet ausgebildetes Eingabemittel, beispielsweise per Tastatur oder mittels eines per Sprachsteuerung arbeitenden Eingabemittels oder über eine direkte Schnittstelle am Lasergerät der Rechnereinheit zuführbar.

Die innerhalb der Rechnereinheit implementierte Auswerteeinheit bestimmt auf der Grundlage der nachfolgenden Formel die sich am Ort der Lichtaustrittsapertur ausbildende Temperatur T in der folgenden Weise:

$$T = \left( 14{,}4\,K * \frac{[Laserleistung\,[W]]}{\left[ Sp\ddot{u}lflussrate\,\left[ \frac{ml}{min} \right] \right]} \right) + Temp.\,d.\,Sp\ddot{u}lfl\ddot{u}ssigkeit\,[K]$$

Die numerische Abhängigkeit der sich während einer laserbasierten Lithotripsie intrakorporal am Ort der Laserlichtapplikation ausbildende Temperatur von der applizierten Laserleistung, der Spülflussrate sowie der Temperatur der Spülflüssigkeit ist in einer großen Vielzahl von in vitro als auch ex vivo durchgeführten Untersuchungen bestätigt worden.

Gleichwohl der vorstehende Algorithmus ein vereinfachter Formelzusammenhang darstellt, lassen sich damit die während der laserbasierten Lithotripsie zu erwartenden intrakorporalen Endtemperaturen am Ort der Steinzertrümmerung hinreichend genau voraussagen, sodass lokale Überhitzungen, die zu irreversiblen Gewebeschädigungen im unmittelbaren Umfeld am Ort der laserbasierten Lithotripsie führen können, ausgeschlossen werden können.

Für den Fall, dass die Lasereinheit keine Schnittstelle besitzt, um den Laserbetrieb anzuzeigen, umfasst die Moduleinheit zusätzlich eine Mikrofoneinheit zur Erkennung eines den Laserbetrieb anzeigenden akustischen Signals, das der Auswerteeinheit zum Zwecke einer zeitsynchronen Signalauswertung zum tatsächlich stattfindenden Laserbetrieb übermittelt wird. So erzeugt der Laser während des Laserbetriebes eindeutig detektierbare akustische Signale, die mit der Mikrofoneinheit zeitaufgelöst erfassbar sind.

Gleichwohl der für die Durchführung der Lithotripsie verantwortliche Arzt der alleinige Entscheidungsträger bei der Durchführung der laserbasierten Lithotripsie ist, kann in einer weiteren Ausführungsform die lösungsgemäß ausgebildete Moduleinheit einen zusätzlichen Notausschalter umfassen, d.h. zusätzlich zu der akustisch, visuell und/oder haptisch in Erscheinung tretenden Signaleinheit, kann das Signal eine Unterbrechung der Stromzuführung zumindest für die Laserquelle auslösen.

In einer weiteren bevorzugten Ausführungsform weist die an eine Endoskopanordnung adaptierbare Moduleinheit zusätzlich eine Einheit zur Erfassung des Füllstandes der innerhalb des Spülflüssigkeitsreservoirs enthaltenen Menge an Spülflüssigkeit auf. Die Einheit ist als am Spülflüssigkeitsreservoir vorgesehener Sensor und/oder in Form eines in der Recheneinheit implementierten, softwarebasierten Auswertealgorithmus ausgebildet, der auf der Grundlage der sensoriell erfassten Spülflussrate und der gemessenen Zeitdauer, während der ein Spülflüssigkeitsaustrag erfolgt, den aktuellen Füllstand ermittelt. Das den Füllstand repräsentierende Sensorsignal bzw. der den Füllstand repräsentierende, numerisch ermittelte Füllstandswert dient zumindest zur Überwachung des Ist-Zustandes sowie einer Prädiktion einer Zeitdauer bis zur vollständigen Entleerung des Spülflüssigkeitsreservoirs oder der Warnung bei Erreichen einer Mindestrestmenge. Die hierdurch gewonnenen Informationen werden durch eine geeignete Anzeige dem behandelnden Arzt optisch oder akustisch wahrnehmbar dargestellt. Die Anzeige kann durch die Signaleinheit erfolgen, über die der die Lithotripsie durchführende Arzt vor möglichen intrakorporalen Überhitzungseffekten gewarnt wird, oder durch eine zusätzliche Anzeige.

Eine weitere Ausführungsform ermöglicht die zusätzliche Überwachung der Temperatur der zugeführten Spülflüssigkeit. Hierzu wird die sensoriell erfasste Temperatur der Spülflüssigkeit (Ts) vorzugsweise als alphanumerischer Wert auf einem Sichtgerät dargestellt. Ferner wird die gemessene Temperatur vorzugsweise der Komparatoreinheit oder einer weiteren Komparatoreinheit zugeführt, wobei bei Unter- oder Überschreiten der Temperatur der Spülflüssigkeit (Ts) unter oder über einen kritischen Temperaturwert ein Signal erzeugt wird, das als Warnung für den behandelnden Arzt dient, bspw. bei einer zu kalten Spülflüssigkeit erfolgt eine Warnung, um die Gefahr einer Unterkühlung für den Patienten zu vermeiden.

In einer weiteren möglichen Ausführungsform sieht die Spülflüssigkeitsfördereinheit ein Mittel vor, durch das die Spülflüssigkeitsrate in Abhängigkeit der sich am Ort der Lichtaustrittapertur ausbildenden Temperatur T und/oder wenigstens eines Betriebsparameters des Lasers regelbar ist. Auf diese Weise eröffnet sich die Möglichkeit einer aktiven Regelung der Spülflüssigkeitsrate der Spülflüssigkeitsfördereinheit in Form einer Pumpe in Abhängigkeit von der applizierten Laserlichtleistung und/oder der berechneten Temperaturen im Gebiet des Eingriffes.

Das lösungsgemäße System zur Temperaturüberwachung bei der Durchführung einer laserlichtbasierten Lithotripsie lässt sich für verschiedene anatomische Bereiche einsetzen, so bspw. im Blasen-, Ureter- oder Nierenbeckenbereich. Je nach Anwendungsbereich kann zwischen verschiedenen Modi zur Anpassung an den Eingriff gewählt werden. Dabei bietet das System die Möglichkeit der Wahl eines Prostata, Harnblasen-, Ureter- oder Nierenbeckenmodus, welche sich durch definierbare Warngrenzen und hinterlegte Kennlinien unterscheiden. Dadurch kann das individuelle Gefahrenspektrum der verschiedenen Operationen adressiert werden.

So lässt sich das System bei allen laserbasierten Verfahren in der Urologie eingesetzt werden, insbesondere bei der Laserlithotripsie, der lasergestützten Prostataenukleation (HoLEP, ThuLEP), der Laservaporisation (PVP) und der interstitiellen Laservaporisation (ILC).)

## Wege zur Ausführung der Erfindung, gewerbliche Verwendbarkeit

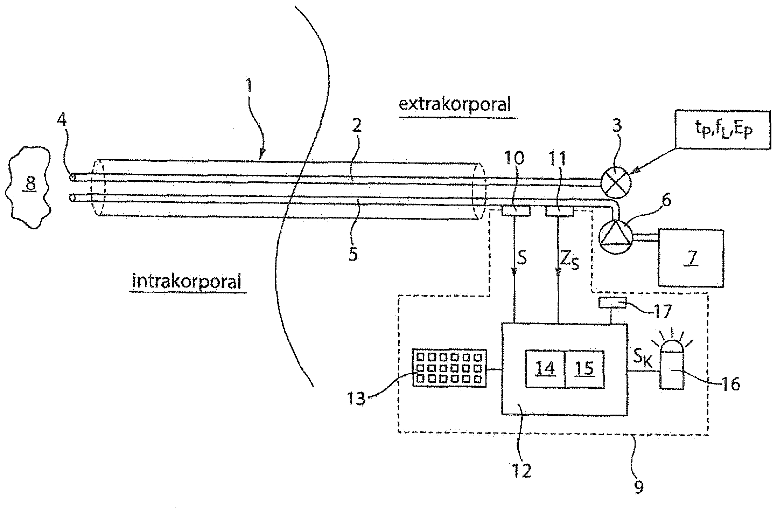

Die einzige Figur zeigt ein Endoskop 1 mit einem Arbeitskanal für einen Lichtleiter 2, der proximalseitig mit einem Laser 3 optisch gekoppelt ist und distalseitig eine Lichtaustrittsapertur 4 aufweist. Ferner verfügt das Endoskop 1 über einen Spülflüssigkeitskanal 5, der im Bereich der Lichtaustrittsapertur 4 distalseitig offen mündet und proximalseitig mit einer Flüssigkeitsfördereinheit 6 sowie einem Flüssigkeitsreservoir 7 fluidisch verbunden ist. Selbstverständlich sind auch Endoskope bekannt, welche den Spülflüssigkeitskanal 5 mit dem Arbeitskanal für den Lichtleiter 2 in einem Kanal vereinen. Derartige an sich bekannte Endoskopanordnungen dienen zur Zertrümmerung intrakorporaler Steine 8 im Wege der laserbasierten Lithotripsie.

Das lösungsgemäße System zur Temperaturüberwachung betrifft eine mit der Endoskopanordnung, umfassend das Endoskop 1, den Laser 3, die Flüssigkeitsfördereinheit 6 sowie das Flüssigkeitsreservoir 7, in dessen extrakorporalen Bereich kombinierbare Moduleinheit 9, die sich einer bevorzugten Ausführungsform aus den folgenden Komponenten zusammensetzt:

Längs der Spülflüssigkeitszuleitung 5 ist ein Durchflusssensor 10, vorzugsweise in Art eines Ultraschallsensors, lösbar fest angebracht. Ebenfalls längs der Spülflüssigkeitskanal 5 oder einer mit diesem fluidische verbundenen Zuleitung ist ein Temperatursensor 11, vorzugsweise in Form eines Infrarotsensors, zur Erfassung der Spülflüssigkeitstemperatur lösbar fest angebracht. Die mittels der Sensoren 10, 11 erzeugten Sensorsignale S, Ts werden einer Rechnereinheit 12 drahtgebunden oder drahtlos übermittelt.

Ferner ist ein Eingabemittel 13 vorgesehen, vorzugsweise in Art einer Schnittstelle zur Lasereinheit zur automatischen Betriebsparameterübergabe, alternativ in Art einer manuell bedienbaren Tastatur, über die die Betriebsparameter des Lasers 3 eingebbar sind. Für den Betrieb des Lasers 3 können die die Laserpulsfrequenz $f_L$ sowie die Einzelpulsenergie $E_P$ durch einen Bediener vorgegeben werden. Eben diese Betriebsparameter sind über das Eingabemittel 13 der Rechnereinheit 12 zuführbar, auf deren Grundlage die am Ort der Lichtaustrittsapertur 4 applizierbare Laserleistung errechnet wird.

Innerhalb der Rechnereinheit 12 ist eine Auswerteeinheit 14 implementiert, die auf der Grundlage der Betriebsparameter des Lasers, der sensorisch erfassten Spülflussrate S sowie der Temperatur Ts der Spülflüssigkeit die sich aktuell am Ort der Lichtaustrittsapertur 4 ausbildende Temperatur unter Zugrundelegung des nachfolgenden Algorithmus ermittelt:

$$T = \left( 14{,}4\,K * \frac{[Laserleistung\,[W]]}{Sp\ddot{u}lflussrate\,\left[\frac{ml}{min}\right]} \right) + Temp.\,d.\,Sp\ddot{u}lfl\ddot{u}ssigkeit\,[K]$$

Die ermittelte intrakorporale Temperatur T wird im Weiteren in einer Komparatoreinheit 15 mit einem einstellbaren Schwellwert verglichen, der vorzugsweise $T_K$ = 42°C beträgt. Im Falle eines Überschreitens des Schwellwertes $T_K$ erzeugt die Komparatoreinheit 15 ein Signal $S_K$, das einer Signaleinheit 16 zugeführt wird, die für den die Lithotripsie durchführenden Arzt haptisch, akustisch und/oder visuell wahrnehmbar in Erscheinung tritt.

Zusätzlich ist eine Mikrofoneinheit 17 mit der Rechnereinheit 12 verbunden, mit der die Aktivität des Lasers 3 erfasst wird, um auf diese Weise sicherzustellen, dass die von Seiten der Rechnereinheit 12 und der darin implementierten Auswerteeinheit 14 verarbeiteten Daten sowie die darauf beruhende Erzeugung eines Signals $S_K$ zeitsynchron mit dem tatsächlichen Betrieb des Lasers 3 sind.

**Bezugszeichenliste**

| | |
|---|---|
| 1 | Endoskop |
| 2 | Lichtleiter |
| 3 | Laser |
| 4 | Lichtaustrittsapertur |
| 5 | Spülflüssigkeitskanal |
| 6 | Spülflüssigkeitsfördereinheit |
| 7 | Spülflüssigkeitsreservoir |
| 8 | Stein |
| 9 | Moduleinheit |
| 10 | Durchflusssensor |
| 11 | Temperatursensor |
| 12 | Rechnereinheit |
| 13 | Eingabemittel |
| 14 | Auswerteeinheit |
| 15 | Komparatoreinheit |
| 16 | Signaleinheit |
| 17 | Mikrofoneinheit |

**Patentansprüche**

1. System zur Temperaturüberwachung bei der Durchführung einer laserlichtbasierten Lithotripsie, bei der eine Endoskopanordnung zum Einsatz kommt, die einen Arbeitskanal für einen Lichtleiter (2), der proximalseitig mit einem Lase (3) optisch gekoppelt ist und distalseitig eine Lichtaustrittsapertur (4) aufweist, sowie einen Spülflüssigkeitskanal (5) umfasst, der im Bereich der Lichtaustrittsapertur distalseitig offen mündet und proximalseitig mit einem Spülflüssigkeitsreservoir (7) fluidisch verbunden ist,
**dadurch gekennzeichnet, dass** das System eine mit der Endoskopanordnung kombinierbare Moduleinheit vorsieht, die folgende Komponenten umfasst:

   einen Durchflusssensor (10), der längs des Spülflüssigkeitskanals und/oder einer mit dem Spülflüssigkeitskanal fluidisch verbundenen Zuleitung anbringbar ist und kontaktfrei zur Spülflüssigkeit eine Spülflussrate bestimmt, ein Eingabemittel, über das Betriebsparameter des Lasers, auf deren Grundlage eine am Ort der Lichtaustrittapertur applizierbare Laserleistung ermittelbar ist, einer mit dem Eingabemittel verbundenen Rechnereinheit übermittelbar sind,
   einen Temperatursensor, der längs des Spülflüssigkeitskanals und/oder einer mit dem Spülflüssigkeitskanal fluidisch verbundenen Zuleitung anbringbar ist und kontaktfrei zur Spülflüssigkeit eine Temperatur der Spülflüssigkeit bestimmt,
   eine in der Rechnereinheit implementierte Auswerteinheit, die zumindest auf der Grundlage der Spülflussrate, der Temperatur der Spülflüssigkeit (Ts) sowie der ermittelten applizierten Laserleistung eine sich während der laserlichtbasierten Lithotripsie intrakorporal am Ort der Lichtaustrittapertur ausbildende Temperatur T numerisch ermittelt, sowie
   eine Komparatoreinheit, die die ermittelte Temperatur T mit einem Schwellwert vergleicht und bei Überschreiten des Schwellwertes ein Signal erzeugt.

2. System nach Anspruch 1,
**dadurch gekennzeichnet, dass** der Durchflusssensor in Art eines Ultraschallsensors ausgebildet ist.

3. System nach Anspruch 1 oder 2,
**dadurch gekennzeichnet, dass** das Eingabemittel eine manuell bedienbare Schnittstelle oder eine Datenschnittstelle ist.

4. System nach einem der Ansprüche 1 bis 3,

**dadurch gekennzeichnet, dass** die Betriebsparameter des Lasers die folgenden vorgebbaren Parameter sind: Laserpulsfrequenz, Einzelpulsenergie, und Laserpulsdauer.

5.   System nach einem der Ansprüche 1 bis 4,
   **dadurch gekennzeichnet, dass** die Auswerteeinheit auf der Grundlage des folgenden Algorithmus die sich am Ort der Lichtaustrittapertur ausbildende Temperatur T bestimmt:

$$T = \left( 14{,}4\,K * \frac{[Laserleistung\ [W]]}{Spülflussrate\ \left[\frac{ml}{min}\right]} \right) + Temp.\,d.\,Spülflüssigkeit\ [K]$$

6.   System nach einem der Ansprüche 1 bis 5,
   **dadurch gekennzeichnet, dass** das System eine physiologisch wahrnehmbare Signaleinheit umfasst, die durch das von der Komparatoreinheit erzeugte Signal haptisch, akustisch und/oder visuell wahrnehmbar in Erscheinung tritt.

7.   System nach einem der Ansprüche 1 bis 6,
   **dadurch gekennzeichnet, dass** eine Kühleinheit vorgesehen ist, die thermisch an den Spülflüssigkeitskanal und/oder an das Spülfluidreservoir ankoppelt, und dass die Kühleinheit durch das von der Komparatoreinheit erzeugte Signal aktivierbar oder steuerbar ist.

8.   System nach einem der Ansprüche 1 bis 7,
   **dadurch gekennzeichnet, dass** eine Notabschaltung vorgesehen ist, die durch das von der Komparatoreinheit erzeugte Signal aktivierbar ist.

9.   System nach einem der Ansprüche 1 bis 8,
   **dadurch gekennzeichnet, dass** das System als Nachrüst-Kit zur Anbringung an eine im Einsatz befindliche Endoskopanordnung ausgebildet ist, die zur Durchführung laserlichtbasierter Lithotripsie geeignet ist.

10.   System nach einem der Ansprüche 1 bis 9,
   **dadurch gekennzeichnet, dass** der Arbeitskanal und der Spülflüssigkeitskanal als ein einheitlicher Kanal ausgebildet sind, so dass der längs des Arbeitskanals angeordnete Lichtleiter von Spülflüssigkeit unmittelbar umspült ist.

11.   System nach einem der Ansprüche 1 bis 10,

   **dadurch gekennzeichnet, dass** die Endoskopanordnung einen extrakorporalen Bereich besitzt, der während der Durchführung der Lithotripsie außerhalb eines Patienten verbleibt, und
   dass der Temperatursensor und der Durchflusssensor der Moduleinheit im extrakorporalen Bereich der Endoskopanordnung längs des Spülflüssigkeitskanals oder einer mit diesem fluidisch verbundenen Zuleitung angebracht sind.

12.   System nach einem der Ansprüche 1 bis 11,

   **dadurch gekennzeichnet, dass** eine Einheit zur Berechnung oder direkten sensorischen Erfassung eines Füllstandes von Spülflüssigkeit in dem Spülflüssigkeitsreservoir vorgesehen ist, und
   dass die Einheit wenigstens ein Signal erzeugt, das vom Füllstand abhängt.

13.   System nach Anspruch 12,
   **dadurch gekennzeichnet, dass** die Einheit ein am Spülflüssigkeitsreservoir vorgesehener Sensor und/oder ein in der Recheneinheit implementierter, softwarebasierter Auswertealgorithmus ist, der auf der Grundlage der sensoriell erfassten Spülflussrate und einer gemessenen Zeitdauer, während der ein Spülflüssigkeitsaustrag erfolgt, den Füllstand ermittelt.

14.   System nach einem der Ansprüche 1 bis 13,

**dadurch gekennzeichnet, dass** die sensoriell erfasste Temperatur der Spülflüssigkeit (Ts) der Komparatoreinheit oder einer weiteren Komparatoreinheit zuführbar ist, die bei Unter- oder Überschreiten der Temperatur der Spülflüssigkeit (Ts) unter oder über einen kritischen Temperaturwert ein Signal erzeugt.

**15.** System nach einem der Ansprüche 1 bis 14,

**dadurch gekennzeichnet, dass** der Spülflüssigkeitskanal proximalseitig mit einer elektrisch betreibbaren Spülflüssigkeitsfördereinheit verbunden ist, und
dass die Spülflüssigkeitsfördereinheit ein Mittel vorsieht, durch das die Spülflüssigkeitsrate in Abhängigkeit der sich am Ort der Lichtaustrittsapertur (4) ausbildenden Temperatur T und/oder wenigstens eines Betriebsparameters des Lasers regelbar ist.

**Claims**

**1.** System for purposes of temperature monitoring when executing a laser light-based lithotripsy, in which an endoscope arrangement is used, which comprises an operating channel for a light guide (2), which on the proximal side is optically coupled to a laser (3), and on the distal side has a light exit aperture (4), together with a flushing fluid channel (5), which on the distal side opens out in the region of the light exit aperture, and on the proximal side is fluidically connected to a flushing fluid reservoir (7),
**characterised in that**, the system provides a modular unit that can be combined with the endoscope arrangement, which modular unit comprises the following components:

a flow rate sensor (10), which can be mounted longitudinally on the flushing fluid channel, and/or on a supply line fluidically connected to the flushing fluid channel, and determines a flushing flow rate without contact with the flushing fluid,
an input means, by way of which, operating parameters of the laser, on the basis of which a laser power can be applied at the location of the light emission aperture, can be determined, can be transmitted to a computer unit connected to the input means,
a temperature sensor, which can be mounted longitudinally on the flushing fluid channel, and/or on a supply line fluidically connected to the flushing fluid channel, and which determines a temperature of the flushing fluid without contact with the flushing fluid,
an evaluation unit implemented in the computer unit, which evaluation unit numerically determines a temperature T forming intracorporeally at the location of the light emission aperture during the laser light-based lithotripsy, at least on the basis of the flushing flow rate, the temperature of the flushing fluid ($T_s$) and the determined applied laser power, together with
a comparator unit, which compares the determined temperature T with a threshold value and generates a signal if the threshold value is exceeded.

**2.** System according to Claim 1,
**characterised in that**, the flow rate sensor is designed in the form of an ultrasonic sensor.

**3.** System according to Claim 1 or 2,
**characterised in that**, the input means is a manually operable interface, or a data interface.

**4.** System according to one of the Claims 1 to 3,
**characterised in that**, the operating parameters of the laser are the following predefinable parameters: laser pulse frequency, single pulse energy, and laser pulse duration.

**5.** System according to one of the Claims 1 to 4,
**characterised in that**, the evaluation unit determines the temperature T forming at the location of the light emission aperture, on the basis of the following algorithm:

$$T = \left( 14.4K * \frac{[Laser\ power\ [W]]}{[Flushing\ flow\ rate\ [ml/min]]} \right) + Flushing\ fluid\ temperature\ [K]$$

**6.** System according to one of the Claims 1 to 5,
**characterised in that**, the system comprises a physiologically perceptible signal unit, which is haptically, acoustically, and/or visually, perceptible by the signal generated by the comparator unit.

**7.** System according to one of the Claims 1 to 6,
**characterised in that**, a cooling unit is provided, which thermally couples to the flushing fluid channel, and/or to the flushing fluid reservoir, and **in that**, the cooling unit can be activated or controlled by the signal generated by the comparator unit.

**8.** System according to one of the Claims 1 to 7,
**characterised in that**, an emergency shut-down is provided, which can be activated by the signal generated by the comparator unit.

**9.** System according to one of the Claims 1 to 8,
**characterised in that**, the system is designed as a retrofit kit for attachment to an endoscope arrangement in use, which is suitable for the execution of laser light-based lithotripsy.

**10.** System according to one of the Claims 1 to 9,
**characterised in that**, the operating channel and the flushing fluid channel are formed as a single channel, so that the light guide arranged longitudinally on the operating channel is directly surrounded by flushing fluid.

**11.** System according to one of the Claims 1 to 10,
**characterised in that**, the endoscope arrangement has an extracorporeal region that remains external to the patient during the performance of lithotripsy, and **in that**, the temperature sensor and the flow rate sensor of the modular unit are mounted in the extracorporeal region of the endoscope arrangement longitudinally on the flushing fluid channel, or on a supply line fluidically connected to the latter.

**12.** System according to one of the Claims 1 to 11,
**characterised in that**, a unit is provided for the calculation, or the direct sensory detection, of a filling level of flushing fluid in the flushing fluid reservoir, and **in that**, the unit generates at least one signal that depends on the filling level.

**13.** System according to Claim 12,
**characterised in that**, the unit is a sensor provided on the flushing fluid reservoir, and/or is a softwarebased evaluation algorithm implemented in the computing unit, which determines the filling level on the basis of the sensor-detected flushing flow rate, and a measured time period, during which a flushing fluid discharge takes place.

**14.** System according to one of the Claims 1 to 13,
**characterised in that**, the temperature of the flushing fluid (Ts), detected by a sensor, can be fed to the comparator unit, or to a further comparator unit, which generates a signal if the temperature of the flushing fluid (Ts) falls below, or exceeds, a critical temperature value.

**15.** System according to one of the Claims 1 to 14,
**characterised in that**, on the proximal side the flushing fluid channel is connected to an electrically operable flushing fluid delivery unit, and **in that**, the flushing fluid delivery unit provides a means by which the flushing fluid rate can be regulated as a function of the temperature T forming at the location of the light emission aperture (4), and/or of at least one operating parameter of the laser.

**Revendications**

**1.** Système, destiné à surveiller la température lors de la réalisation d'une lithotritie endo-corporelle à la lumière laser, lors de laquelle est mis en œuvre un agencement endoscopique, qui comporte un canal de travail pour un conducteur optique (2), qui du côté proximal est optiquement connecté avec un laser (3) et du côté distal comprend une ouverture (4) de sortie de la lumière, ainsi qu'une canalisation (5) de liquide de lavage, qui débouche ouvertement du côté distal dans la zone de l'ouverture de sortie de la lumière et qui est fluidiquement reliée du côté proximal avec un réservoir (7) de liquide de lavage,
**caractérisé en ce que** le système prévoit une unité modulaire, susceptible d'être associée avec l'agencement endoscopique, qui comprend les composants suivants :

un débitmètre (10), qui est susceptible d'être monté le long de la canalisation de liquide de lavage et / ou d'une conduite d'arrivée fluidiquement reliée avec la canalisation de liquide de lavage et qui, sans contact avec le liquide de lavage détermine un débit du flux de lavage,

un moyen de saisie, par l'intermédiaire duquel des paramètres de service du laser, sur la base desquels une puissance du laser applicable à l'endroit de l'ouverture de sortie de lumière peut être déterminée sont transmissibles à une unité de calcul connectée avec le moyen de saisie,

un capteur de température, qui est susceptible d'être monté le long de la canalisation de liquide de lavage et / ou d'une conduite d'arrivée fluidiquement reliée avec la canalisation de liquide de lavage et qui, sans contact avec le liquide de lavage détermine une température du liquide de lavage,

une unité d'évaluation implémentée dans l'unité de calcul, qui au moins sur la base du débit du flux de lavage, de la température du liquide de lavage ($T_s$), ainsi que de la puissance appliquée du laser déterminée, détermine numériquement une température T qui se créé de manière intracorporelle à l'endroit de l'ouverture de sortie de la lumière pendant la lithotritie à la lumière laser, ainsi

qu'une unité de comparaison, qui compare la température T déterminée avec une valeur seuil et lors du dépassement de la valeur seuil, génère un signal.

2. Système selon la revendication 1,
**caractérisé en ce que** le débitmètre est conçu à la manière d'un capteur ultrasonique.

3. Système selon la revendication 1 ou 2,
**caractérisé en ce que** le moyen de saisie est une interface à commande manuelle ou une interface de données.

4. Système selon l'une quelconque des revendications 1 à 3,
**caractérisé en ce que** les paramètres de service du laser sont les paramètres prédéfinissables suivants : la fréquence d'impulsion du laser, l'énergie d'une impulsion individuelle et la durée d'impulsion du laser.

5. Système selon l'une quelconque des revendications 1 à 4,
**caractérisé en ce que** l'unité d'évaluation détermine la température T qui se créé à l'endroit de l'ouverture de sortie de la lumière sur la base de l'algorithme suivant :

$$T = \left( 14{,}4\ K * \frac{puissance\ du\ laser\ [W]}{débit\ du\ flux\ de\ lavage\ \left[\frac{ml}{min}\right]} \right) + temp.\ du\ liquide\ de\ lavage\ [K]$$

6. Système selon l'une quelconque des revendications 1 à 5,
**caractérisé en ce que** le système comprend une unité de signalisation physiologiquement perceptible, qui apparaît de manière perceptible par voie haptique, acoustique et / ou visuelle, du fait du signal généré par l'unité d'évaluation.

7. Système selon l'une quelconque des revendications 1 à 6,
**caractérisé en ce qu'**il est prévu une unité de refroidissement qui s'accouple thermiquement sur la canalisation de liquide de lavage et / ou sur le réservoir de fluide de lavage et **en ce que** l'unité de refroidissement est susceptible d'être activée ou commandée par le signal généré par l'unité de comparaison.

8. Système selon l'une quelconque des revendications 1 à 7,
**caractérisé en ce qu'**il est prévu un arrêt d'urgence, qui est susceptible d'être activé par le signal généré par l'unité de comparaison.

9. Système selon l'une quelconque des revendications 1 à 8,
**caractérisé en ce que** le système est conçu sous la forme d'un kit d'extension, destiné à être monté sur un agencement endoscopique en service, qui est apte à réaliser une lithotritie à la lumière laser.

10. Système selon l'une quelconque des revendications 1 à 9,
**caractérisé en ce que** la canalisation de travail et la canalisation de liquide de lavage sont conçues sous la forme d'une canalisation homogène, de sorte que le conducteur optique placé le long de la canalisation de travail soit directement baigné dans le liquide de lavage.

11. Système selon l'une quelconque des revendications 1 à 10,

**caractérisé en ce que** l'agencement endoscopique détient une zone extracorporelle, qui pendant la réalisation de la lithotritie reste à l'extérieur d'un patient, et **en ce que** le capteur de température et le débitmètre de l'unité modulaire sont montés dans la zone extracorporelle de l'agencement endoscopique, le long de la canalisation de liquide de lavage ou d'une conduite d'arrivée fluidiquement reliée avec celle-ci.

12. Système selon l'une quelconque des revendications 1 à 11,
    **caractérisé en ce qu'**il est prévu une unité destinée au calcul ou à la détection sensorielle d'un niveau de remplissage du liquide de lavage dans le réservoir de liquide de lavage, et **en ce que** l'unité génère au moins un signal qui dépend du niveau de remplissage.

13. Système selon la revendication 12,
    **caractérisé en ce que** l'unité est un capteur prévu sur le réservoir de liquide de lavage et / ou un algorithme d'évaluation logiciel, implémenté dans l'unité de calcul, qui détermine le niveau de remplissage sur la base du débit du flux de lavage détecté de manière sensorielle et d'une durée mesurée, pendant laquelle a lieu l'évacuation de liquide de lavage.

14. Système selon l'une quelconque des revendications 1 à 13,
    **caractérisé en ce que** la température du liquide de lavage ($T_s$) détectée de manière sensorielle peut être amenée vers l'unité de comparaison ou une unité de comparaison supplémentaire, qui lors d'une non-atteinte ou d'un dépassement de la température du liquide de lavage ($T_s$) sous ou au-dessus d'une valeur de température critique génère un signal.

15. Système selon l'une quelconque des revendications 1 à 14,
    **caractérisé en ce que** la canalisation de liquide de lavage est reliée du côté proximal avec une unité de transport de liquide de lavage à actionnement électrique et **en ce que** l'unité de transport de liquide de lavage prévoit un moyen permettant de réguler le débit de liquide de lavage en fonction de la température T qui se créé à l'endroit de l'ouverture de sortie de la lumière (4) et / ou d'au moins un paramètre de service du laser.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20180055568 A1
- DE 202017102316 U1